# EUROPEAN PATENT APPLICATION

(11) **EP 2 548 559 A1**
(43) Date of publication of application: **23.01.2013**
(21) Application number: 12188643.6
(22) Date of filing: 11.12.2007
(51) Int. Cl.: A61K 31/559, A61P 1/04

(54) **Methods and compositions for treating gastrointestinal disorders**

(30) Priority: 18.12.2006 US 870444 P
(62) Divisional of application: 07855057.1
(71) Applicant: Allergan, Inc., Irvine, CA 92612 (US)
(72) Inventor: Jiang, Guang Liang, Irvine, CA California 92620 (US); Im, Wha Bin, Irvine, CA California 92612 (US); Wheeler, Larry A., Irvine, CA California 92612 (US)
(74) Representative: HOFFMANN EITLE

(57) **Abstract**

Methods are provided directed to administering a therapeutically effective amount of a prostaglandin EP4 agonist component to a mammal afflicted with or prone to affliction with a disease or condition selected from an esophageal ulcer, alcohol gastropathy, a duodenal ulcer, a gastric ulcer, non-steroidal anti- inflammatory drug-induced gastroenteropathy and intestinal ischemia. Such administration results in treating or preventing the disease or condition.

## Description

### CROSS-REFERENCE

This application claims the benefit of U.S. Provisional Application serial number 60/870,444, filed December 18, 2006, which is hereby incorporated by reference in its entirety.

### FIELD OF THE INVENTION

This invention relates to treating or preventing certain diseases or conditions using therapeutically active compositions. Particularly, this invention relates to methods using certain prostaglandin EP₄ agonist components to treat or prevent certain diseases or conditions of the gastrointestinal tract, including, without limitation, those conditions having an effect on the esophagus, stomach, small intestine, and/or large intestine.

### BACKGROUND OF THE INVENTION

Prostaglandins potentiate members of a subfamily of the G-protein-coupled receptor (GPCR) family of cell surface receptors. Members of the GPCR family of receptors are characterized by being folded and configured in such a manner as to cross the cell membrane seven times; thus, these receptors are also sometimes referred to as seven-transmembrane receptors.

There are currently nine known receptors of prostaglandins in various cell types. These receptors are termed DP1-2, EP1-4, FP, IP, and TP, with the nomenclature corresponding to the receptor that binds and is potentiated by the corresponding prostaglandin (e.g., EP₄ receptors bind to and are potentiated by prostaglandin E₄ or PGE₄). Prostaglandins thus act on a variety of cells such as vascular smooth muscle cells causing constriction or dilation, on platelets causing aggregation or disaggregation and on spinal neurons causing pain. Prostaglandins have a wide variety of actions, including, but not limited to muscular constriction and the mediation of inflammation. Other effects include calcium movement, hormone regulation and cell growth control. Thromboxane is created in platelets and causes vascular constriction and platelet aggregation. Prostacyclin is synthesized by cells in the blood vessel walls and is antagonistic to thromboxane.

Prostaglandins can be described with reference to their structural similarity to prostanoic acid, and have the following structural formula and numbering scheme:

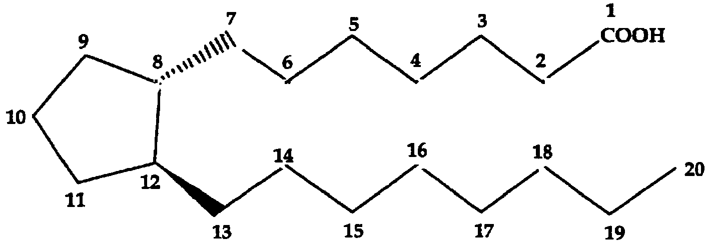

Various types of prostaglandins are known, depending on the structure of and substituents carried on the alicyclic ring of the prostanoic acid skeleton. Further classification of prostaglandins is based on the number of unsaturated bonds in the side chain indicated by numerical subscripts after the generic type of prostaglandin [e.g. prostaglandin E₁ (PGE₁), prostaglandin E₂ (PGE₂)], and on the configuration of the substituents on the alicyclic ring indicated by α or β [e.g. prostaglandin F_{2α} (PGF₂β)].

In addition to the naturally occurring prostaglandins, various non-naturally occurring analogs, derivatives, and other compounds having some structural similarity to the prostaglandins have also been discovered to have activity at the prostaglandin receptors in general, and the EP₄ receptor in particular.

A compound of particular interest that has prostaglandin EP₄ receptor agonist activity has the chemical name (Z)-7-{(1R,4S,5R)-5-[(E)-5-(3-chloro-benzo[b]thiophene-2-yl)-3-hydroxy-pent-1-enyl]-4-hydroxy-3,3-dimethyl-2-oxo-cyclopentyl}-hept-5-enoic acid, and is disclosed in U.S. Patent Publication No. 2005/0164992, hereby incorporated by reference herein.

Prostaglandin EP₄ receptor selective agonists are believed to have several medical uses. For example, U.S. Patent No. 6,552,067 B2, expressly incorporated by reference herein, teaches the use of prostaglandin EP₄ receptor selective agonists for the treatment of "methods of treating conditions which present with low bone mass, particularly osteoporosis, frailty, an osteoporotic fracture, a bone defect, childhood idiopathic bone loss, alveolar bone loss, mandibular bone loss, bone fracture, osteotomy, bone loss associated with periodontitis, or prosthetic ingrowth in a mammal."

U.S. Patent No. 6, 586, 468 B1, expressly incorporated by reference herein, teaches that certain prostaglandin EP₄ receptor selective agonists "are useful for the prophylaxis and/or treatment of immune diseases (autoimmune diseases (amyotrophic lateral sclerosis (ALS), multiple sclerosis, Sjoegren's syndrome, arthritis, rheumatoid arthritis, systemic lupus erythematosus, etc.), post-transplantation graft rejection, etc.), asthma, abnormal bone formation, neurocyte death, pulmopathy, hepatopathy, acute hepatitis, nephritis, renal insufficiency, hypertension, myocardial ischemia, systemic inflammatory syndrome, pain induced by ambustion, sepsis, hemophagocytosis syndrome, macrophage activation syndrome, Still's diseases, Kawasaki diseases, burns, systemic granuloma, ulcerative colititis, Crohn's diseases, hypercytokinemia at dialysis, multiple organ failure, shock, etc."

U.S. Patent Publication No. 2005/0164992, incorporated by reference above and commonly owned by the current assignee discloses the use of certain prostaglandin EP₄ receptor agonists for the treatment of inflammatory bowel disease (IBD), characterized by inflammation in the large or small intestines and is manifest in symptoms such as diarrhea, pain, and weight loss. Nonsteroidal anti-inflammatory drugs are thought to be associated with the risk of developing IBD, and Kabashima and colleagues have disclosed that "EP₄ works to keep mucosal integrity, to suppress the innate immunity, and to downregulate the proliferation and activation of CD4+ T cells. These findings have not only elucidated the mechanisms of IBD by NSAIDs, but also indicated the therapeutic potential of EP₄-selective agonists in prevention and treatment of IBD." (Kabashima, et. al., The Journal of Clinical Investigation, April 2002, Vol. 9, 883-893).

Various other conditions of the gastrointestinal tract may occur to the detriment of the individual affected. Among such diseases or conditions are gastroesophageal reflux disease, acute and chronic gastritis (including, but not limited to *Heliobacter* pylori-induced gastritis, atrophic gastritis, pernicious anemia, stress-related mucosal damage, alcohol gastropathy, postoperative alkaline gastritis, and eosinophilic gastroenteritis), duodenitis, diverticulitis, Zolliger-Ellison syndrome, chmotherepy-induced gastrointestinal toxicity, radiation-induced gastrointestinal toxicity, and wounds or damage to the gastrointestinal tract caused by injury or surgery.

New methods and compositions for treating or preventing such diseases or conditions would be highly beneficial.

### SUMMARY OF THE INVENTION

The present invention relates to methods and compositions for treating or preventing one or more diseases or conditions of the gastrointestinal tract of for example, the mammalian body. In particular, the present invention relates to methods and compositions for treating gastrointestinal conditions including gastroesophageal reflux disease, acute and chronic gastritis (including, but not limited to Heliobacter pylori-induced gastritis, atrophic gastritis, pernicious anemia, stress-related mucosal damage, alcohol gastropathy, postoperative alkaline gastritis, and eosinophilic gastroenteritis), duodenitis, diverticulitis, Zolliger-Ellison syndrome, chemotherapy-induced gastrointestinal toxicity, radiation-induced gastrointestinal toxicity, and wounds or damage to the gastrointestinal tract caused by injury or surgery. Treating or preventing such disease(s) or condition(s) provides one or more substantial advantages, for example, enhances or maintains the health of the individual, for example, human or animal, afflicted with or prone to affliction with such disease(s) or condition(s).

In one broad aspect of the invention, the present invention is drawn to methods comprise administering a therapeutically effective amount of a therapeutically effective composition comprising a prostaglandin EP₄ agonist having a structure as follows (and salts and esters thereof, as well as mixtures of these): 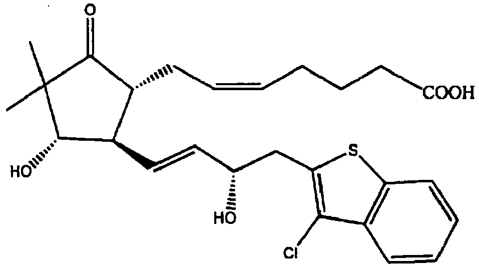 to a mammal afflicted with or prone to affliction with one or more diseases or conditions selected from gastroesophageal reflux disease, acute and chronic gastritis (including, but not limited to *Heliobacter* pylori-induced gastritis, atrophic gastritis, pernicious anemia, stress-related mucosal damage, alcohol gastropathy, postoperative alkaline gastritis, and eosinophilic gastroenteritis), duodenitis, diverticulitis, Zolliger-Ellison syndrome, chemotherapy-induced gastrointestinal toxicity, radiation-induced gastrointestinal toxicity, and wounds or damage to the gastrointestinal tract caused by injury or surgery, thereby treating or preventing the one or more diseases or conditions.

In another embodiment, the invention is drawn to pharmacologically effective compositions comprising a prostaglandin EP₄ agonist having a structure as follows (and salts and esters thereof, as well as mixtures of these):

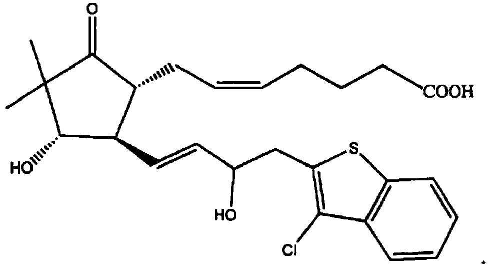

In another embodiment, the therapeutically effective composition is administered to a human. The prostaglandin EP₄ agonist component may be administered, for example, orally administered, to the gastrointestinal tract of a mammal, for example, a human.

Any and all features described herein and combinations of such features are included within the scope of the present invention provided that the features of any such combination are not mutually inconsistent.

### BRIEF DESCRIPTION OF THE DRAWINGS

Fig. 1A shows a Western blot of a polyacrylamide electrophoresis gel of an IEC-18 cell lysate having bands for the Akt protein, in which an antibody selective for phophorylated Akt has been secondarily used as a probe of Akt species. Lanes include samples from: cells treated with vehicle (0.1% DMSO) alone, cells treated with 10 µM celecoxib, cells treated with 5 µM SC-560, and cells treated with 10 µM indomethacin.
Fig 1B shows a Western blot of a polyacrylamide electrophoresis gel of an IEC-18 cell lysate having bands for the Akt protein, in which an antibody selective for phophorylated Akt has been secondarily used as a probe of Akt species. Lanes include samples from: vehicle-treated cells, cells treated with indomethacin (10 µM)alone, and cells treated with indomethacin and 10 nM Compound 7.
Fig. 2 shows flow cytometry (FACS) scans of IEC-18 cells treated with vehicle alone (Fig. 2A), cells treated with both doxorubicin (0.05 µg/ml) and celecoxib (10 µM)(Fig. 2B), and cells treated with both doxorubicin and celecoxib and 10 nM Compound 7 (Fig. 2C) .
Fig. 2D is a bar graph showing the relative degrees of apoptosis in these samples.
Fig. 3A is a bar graph showing ulcer sizes in a mouse ulcer model, wherein non-control mice were given indomethacin.
Fig. 3B is a bar graph showing white blood cells, lymphocytes, neutrophils and monocyte cell counts in a mouse ulcer model, wherein non-control mice were given indomethacin.
Fig. 3C is a bar graph showing red blood cell, hemoglobin (HGB) and hematocrit levels in a mouse ulcer model, wherein non-control mice were given indomethacin.
Fig. 4A is a bar graph showing ulcer sizes in a mouse ulcer model, wherein control mice were given vehicle and non-control mice were given Compound 7.
Fig. 4B is a bar graph showing neutrophil and necrosis levels in granulation tissue in a mouse ulcer model, wherein control mice were given vehicle and non-control mice were given Compound 7.
Figure 4C shows photographs comparing gross and microscopic morphologies of ulcer tissue in a mouse ulcer model, wherein control mice were given vehicle and non-control mice were given Compound 7.
Fig. 5A is a bar graph showing red blood cell, hemoglobin (HGB) and hematocrit levels in a mouse ulcer model, wherein control mice were given indomethacin (3 mg/kg/d) and non-control mice were given indomethacin (3 mg/kg/d) and Compound 7 (0.1 mg/kg/d).
Figure 5B is a bar graph showing white blood cell (WBC), lymphocyte (LYM) and neutrophil (NEU) levels in a mouse ulcer model, wherein control mice were given indomethacin (3 mg/kg/d) and non-control mice were given indomethacin and Compound 7 (0.1 mg/kg/d).

### DETAILED DESCRIPTION

A prostaglandin EP₄ agonist is broadly defined as a compound that the person of ordinary skill in the art reasonably believes agonizes a prostaglandin EP₄ receptor according to any one or more of numerous assays for determination of the EP₄ activity that are well known to such persons. For example, and without limitation, one such assay system is known as the RSAT assay technology disclosed in part in Brann, U.S. Patent No. 5,707,798, hereby incorporated herein by reference. This assay system is commonly used to assay the effects (rather than simply the binding) of putative modulators of members of the G-protein coupled receptor (GPCR) superfamily of cell surface receptors at a given cloned receptor. This GPCR superfamily includes the prostaglandin EP₄ receptor.

Other assays that may be used to determine the activity of a modulator of the prostaglandin EP4 receptor include employing cells expressing and displaying recombinant EP4 receptor in an assay of intracellular calcium signaling. When cells are preloaded with a fluorescent dye, such as the Fluo-4^{®} dye, the subsequent release of Ca⁺⁺ from intracellular depots is detected as an increase in fluorescence at the emission maximum of Fluo-4^{®} (between 510 and 570 nm). Prostaglandin EP₄ agonist activity can therefore be measured by a change in intracellular calcium concentration using a device such as the FLIPR^{®} apparatus.

Prodrugs of the prostaglandin EP₄ agonists disclosed and used in U.S. Patent Publication No. 2005/0164992, incorporated by reference in its entirety as part of this specification above and commonly owned by the current assignee, are contemplated to be within the scope of the methods and compositions of present invention as they may apply to the particular EP₄ receptor agonists disclosed herein. Thus, it will be understood that the EP4 agonist compounds disclosed herein may be administered as therapeutic agents as the biologically active compound or as one or more prodrug, for example and without limitation, those derived from or containing alkene, alkyne and ester linkages; an ester, ether, or amide of a carbohydrate; an ester, ether, or amide of an amino acid; a glucoside ester, ether, or amide; a glucuronide ester, ether, or amide; a cyclodextrin ester, ether, or amide; or a dextran ester, ether, or amide, and mixtures thereof.

In general, the formulations and methods for delivering a drug to the gastrointestinal tract, or desired portion thereof, are well known in the art. See, e.g., REMINGTON'S PHARMACEUTICAL SCIENCES Mack Publishing Company, Easton, Pa. 16th Edition (1980). For example, oral dosage forms may include solid forms and semi-solid forms (such as tablets, hard and soft capsules, including liquid or gel filled capsules or tablets), and aqueous and non-aqueous liquid forms, including but not limited to, oil-in-water and water-in-oil emulsions, liquid suspensions, solutions, syrups and the like, are known in the art.

Specific formulations of oral dosage forms include, without limitation, 1) contacting the drug with compatible excipients, for example, conventional excipients, including, without limitation, oils, such as hydrogenated castor oil other vegetable oils, and the like and mixtures thereof; cellulosic derivatives and starch derivatives, such as alkyl celluloses, hydroxyl alkyl celluloses, alkali metal starch carboxylates, e.g., sodium starch glycolate, and the like and mixtures thereof; and sugars and sugar derivatives and the like and mixtures thereof; so that the drug is released in the upper or lower gastrointestinal tract, for example, esophagus, stomach, duodenum and colon. 2) contacting a prodrug with compatible excipients, for example the conventional excipients noted in 1) above, with the prodrug being selected so that the drug is released in the upper gastrointestinal tract and/or lower gastrointestinal tract, as desired, 3) coating the drug and/or prodrug with, or encapsulating or impregnating the drug and/or prodrug into, a polymer such as a alkyl cellulose or derivative thereof and/or gelatin designed for delivery to the upper and or lower gastrointestinal tract, 4) formulations for time released delivery of the drug and/or prodrug, 5) use of a bioadhesive system, such as a transdermal patch and the like.

If desired, the presently useful compositions or dosage forms may additionally comprise other pharmaceutically acceptable excipients, such as tonicity components, buffer components, electrolyte components, thickeners, fillers, diluents, flavoring agents, coloring agents, antioxidants, preservatives (such as antibacterial or antifungal agents), acids and/or bases to adjust pH, and the like and mixtures thereof. Each such additive, if present, may typically comprise about 0.0001% or less or about 0.01% or less to about 10% or more by weight of the composition. Such additives may include those additives which are conventional and/or well known for use in similar pharmaceutical compositions. For example, suitable thickening agents include any of those known in the art, as for example pharmaceutically acceptable polymers and/or inorganic thickeners. Such agents include, but are not limited to, polyacrylate homo- and co-polymers; celluloses and cellulose derivatives; polyvinyl pyrrolidones; polyvinyl resins; silicates; and the like and mixtures thereof.

In one embodiment, the use of an azo-based prodrug may be employed to provide the drug in the lower gastrointestinal tract. Lower intestinal microflora are believed to be capable of reductive cleavage of an azo bond leaving the two nitrogen atoms as amine functional groups. Bacteria of the lower gastrointestinal tract also have enzymes which can digest glycosides, glucuronides, cyclodextrins, dextrans, and other carbohydrates, and ester prodrugs formed from these carbohydrates have been shown to deliver the parent active drugs selectively to the lower gastrointestinal tract.

Carbohydrate polymers including, without limitation, amylase, arabinogalactan, chitosan, chondroiton sulfate, dextran, guar gum, pectin, xylin, and the like and mixtures thereof, can be used to coat a drug and/or prodrug, or a drug and/or prodrug may be impregnated or encapsulated in the polymer. After oral administration, the polymers remain stable in the upper gastrointestinal tract, but are digested by the microflora of the lower gastrointestinal tract thus releasing the drug for therapeutic effect.

Polymers that are sensitive to pH may also be used since the lower gastrointestinal tract has a higher pH than the upper gastrointestinal tract. Such polymers are commercially available. For example, Rohm Pharmaceuticals, Darmstadt, Germany, markets pH dependent methacrylate based polymers and copolymers sold under the trademark Eudragit®, which have varying solubilities over different pH ranges based upon the number of free carboxylate groups in the polymer. Time release systems, bioadhesive systems, and other delivery systems may also be employed.

Coadministration of prostaglandin EP₄ agonists with one or more other drugs, such as drugs other than EP4 agonists, either in a single composition or in separate dosage forms, is also contemplated. While not intending to limit the scope of the invention in any way, other drugs which may be included in combination therapies aimed at treating gastrointestinal disorders with prostaglandin EP₄ agonists and their prodrugs include, but are not limited to:
Anti-inflammatory drugs, such as non-selective COX inhibitors and selective COX-2 inhibitors including without limitation, aspirin and aspirin derivatives, acetomenifen, ibuprofen, ketorolac, napoxen, piroxicam, nabumetone, diclofenac, diflunisal, etodolac, fenoprofen, ketoprofen, mefenamic acid, meloxicam, nabumetone, oxaprozin, sulindac, and tolmetin and the like and mixtures thereof; indoles, such as indomethacin and the like; diarylpyrazoles, such as celecoxib and the like; pyrrolo pyrroles; other agents that inhibit prostaglandin synthesis; aminosalicylates; other non-steroidal anti-inflammatory drugs, and the like and prodrugs and mixtures thereof;
Steroids, such as hydrocortisone, cortisone, prednisolone, prednisone, triamcinolone, dexamethasone, medrysone, fluorometholone, estrogens, progesterones, and the like and mixtures thereof;
Immunomodulators, such as azathioprine, 6-mercaptopurine, cyclosporine (such as cyclosporine A), and the like and mixtures thereof; and Humanized and non-humanized monoclonal antibodies (including fragments, derivatives and muteins thereof) against pro-inflammatory cytokines, such as infliximab, etanercept, onercept, adalimumab, CDP571, CDP870, natalizumab, MLN-02, ISIS 2302, cM-T412, BF-5, vasilizumab, daclizumab, ranibizymab, bevacizumab, basiliximab, Anti-CD40L, and the like and mixtures thereof.

Such other drug or drugs are administered in amounts effective to provide the desired therapeutic effect or effects.

Assays for screening and determining prostaglandin EP₄ agonist activity and selectivity are described below, but are not the only such assays for this purpose known to those of skill in the art.

### HUMAN RECOMBINANT EP₁, EP₂, EP₃, EP₄, FP, TP, IP and DP RECEPTORS: STABLE TRANSFECTANTS

Plasmids encoding the human EP₁, EP₂, EP₃, EP₄, FP, TP, IP and DP receptors are prepared by cloning the respective coding sequences into the eukaryotic expression vector pCEP₄ (Invitrogen). (The pCEP₄ vector contains an Epstein-Barr virus (EBV) origin of replication, which permits episomal replication in primate cell lines expressing EBV nuclear antigen (EBNA-1). It also contains a hygromycin resistance gene that is used for eukaryotic selection. The cells employed for stable transfection are human embryonic kidney cells (HEK-293) that are transfected with and express the EBNA-1 protein. These HEK-293-EBNA cells (Invitrogen) are grown in medium containing Geneticin (G418) to maintain expression of the EBNA-1 protein. HEK-293 cells are grown in DMEM with 10% fetal bovine serum (FBS), 250 µg ml⁻¹ G418 (Life Technologies) and 200 µg ml⁻¹ gentamicin or penicillin/streptomycin. Selection of stable transfectants is achieved with 200 µg ml⁻¹ hygromycin, the optimal concentration being determined by previous hygromycin kill curve studies.

For transfection, the cells are grown to 50-60% confluency on 10 cm plates. The plasmid pCEP₄ incorporating cDNA inserts for the respective human prostanoid receptor (20 µg) is added to 500 µl of 250 mM CaCl₂. HEPES buffered saline x 2 (2 x HBS, 280 mM NaCl, 20 mM HEPES acid, 1.5 mM Na₂ HPO₄, pH 7.05 - 7.12) is then added dropwise to a total of 500 µl, with continuous vortexing at room temperature. After 30 min, 9 ml DMEM are added to the mixture. The DNA/DMEM/calcium phosphate mixture is then added to the cells, which is previously rinsed with 10 ml PBS. The cells are then incubated for 5 hr at 37°C in humidified 95% air/5% CO₂. The calcium phosphate solution is then removed and the cells are treated with 10% glycerol in DMEM for 2 min. The glycerol solution is then replaced by DMEM with 10% FBS. The cells are incubated overnight and the medium is replaced by DMEM/10% FBS containing 250 µg ml⁻¹ G418 and penicillin/streptomycin. The following day hygromycin B is added to a final concentration of 200 µg ml⁻¹.

Ten days after transfection, hygromycin B resistant clones are individually selected and transferred to a separate well on a 24 well plate. At confluence each clone is transferred to one well of a 6 well plate, and then expanded in a 10 cm dish. Cells are maintained under continuous hygromycin selection until use.

### RADIOLIGAND BINDING

Radioligand binding studies are a useful initial step in screening for prospective modulators of the prostaglandin EP receptor; however, binding studies alone can give no information concerning whether a binding ligand is an agonist, inverse agonist, or antagonist of the receptor. Radioligand binding studies on plasma membrane fractions prepared from cells are performed as follows. Cells washed with TME buffer are scraped from the bottom of the flasks and homogenized for 30 sec using a Brinkman PT 10/35 polytron. TME buffer is added as necessary to achieve a 40 ml volume in the centrifuge tubes. TME is comprised of 50 mM TRIS base, 10 mM MgCl₂, 1 mM EDTA; pH 7.4 is achieved by adding 1 N HCl The cell homogenate is centrifuged at 19,000 rpm for 20-25 min at 4°C using a Beckman Ti-60 or Ti-70 rotor. The pellet is then resuspended in TME buffer to provide a final protein concentration of 1 mg/ml, as determined by Bio-Rad assay. Radioligand binding assays are performed in a 100 µl or 200 µl volume.

The binding of [³H] PGE₂ (specific activity 165 Ci/mmol) is determined in duplicate and in at least 3 separate experiments. Incubations are for 60 min at 25° C and are terminated by the addition of 4 ml of ice-cold 50 mM TRIS-HCl followed by rapid filtration through Whatman GF/B filters and three additional 4 ml washes in a cell harvester (Brandel). Competition studies are performed using a final concentration of 2.5 or 5 nM [³H] PGE₂ and non-specific binding is determined with 10⁻⁵ M unlabelled PGE₂.

For all radioligand binding studies, the criteria for inclusion are >50% specific binding and between 500 and 1000 displaceable counts or better.

### Methods for FLIPR^{®} (Fluorometric Imaging Plate Reader) Studies

### (a) Cell Culture

HEK-293 (EBNA) cells, stably expressing one type or subtype of recombinant human prostaglandin receptors (prostaglandin receptors expressed: hDP/Gqs5; hEP.₁ ; hEP₂ /Gqs5; hEP_{3A} /Gqi5; hEP₄ /Gqs5; hFP; hIP; hTP), are cultured in 100 mm culture dishes in high-glucose DMEM medium containing 10% fetal bovine serum, 2 mM 1-glutamine, 250 µg/ml geneticin (G418) and 200 µg/ml hygromycin B as selection markers, and 100 units/ml penicillin G, 100 µg/ml streptomycin and 0.25 µg/ml amphotericin B.

### (b) Calcium Signal Studies on the FLIPR^{®}.

Transfectant cells are seeded at a density of 5 x10⁴ cells per well in Biocoat* .Poly-D-lysine-coated black-wall, clear-bottom 96-well plates (Becton-Dickinson) and allowed to attach overnight in an incubator at 37°C. Cells are then washed two times with HBSS-HEPES buffer (Hanks Balanced Salt Solution without bicarbonate and phenol red, 20 mM HEPES, pH 7.4) using a Denley Cellwash plate washer (Labsystems). After 45 minutes of dye-loading in the dark, using the calcium-sensitive dye Fluo-4 AM at a final concentration of 2 µM, plates are washed four times with HBSS-HEPES buffer to remove excess dye leaving 100 µl in each well. Plates are then re-equilibrated to 37°C. for a few minutes.

Cells are excited with an Argon laser at 488 nm, and emission measured through a 510-570 nm bandwidth emission filter (FLIPR.TM., Molecular Devices, Sunnyvale, Calif.). Drug solution is added in a 50 µl volume to each well to give the desired final concentration. The peak increase in fluorescence intensity is recorded for each well. On each plate, four wells each serve as negative (HBSS-HEPES buffer) and positive controls (standard agonists: BW245C (hDP); PGE₂ (hEP₁ ; hEP₂ /Gqs5; hEP_{3A} /Gqi5; hEP₄ /Gqs5); PGF_{2α}; (hFP); carbacyclin (hIP); U-46619 (hTP), depending on receptor). The peak fluorescence change in each drug-containing well is then expressed relative to the controls.

Compounds are tested in a high-throughput (ETS) or concentration-response (CoRe) format. In the HTS format, forty-four compounds per plate are examined in duplicates at a concentration of 10⁻⁵ M. To generate concentration-response curves, four compounds per plate are tested in duplicates in a concentration range between 10⁻⁵ and 10⁻¹¹ M. The duplicate values are averaged. In either, HTS or CoRe format each compound are tested on at least 3 separate plates using cells from different passages to give an n value of 3.

### MEASUREMENT OF CHARGES IN INTRACELLULAR CYCLIC AMP

Other assays of c-protein coupled receptor agonist activity may employ the detection of changes in the concentrations of intracellular second messengers other than Ca++, such as cyclic AMP (cAMP). cAMP assay methods are very well known. Such methods may include, for example, the cotransfection of nucleic acids encoding the receptor, such as the prostaglandin EP₄ receptor aderenergic receptor, with a cAMP-dependent chloramphenicol acetyl transferase (CAT) reporter plasmid into human JEG-3 choriocarcinoma cells, and challenging the cells with modulators of the receptor. For example, human JEG-3 cells (American Type Culture Collection, Rockville, Md.) are cultured in Delbecco's Modified Eagle's Medium (DMEM) containing 10% fetal calf serum (FCS), 100 units/ml penicillin, and 100 micrograms/ml streptomycin. Cells are plated in 10 cm dishes 1-2 days before transfection. Cells are then transfected with 10 micrograms of a CAT reporter such as plasmid TESBglIICRE(+)Δ NHSE (provided by P. Mellon, Salk Institute, La Jolla, Calif.) containing an 18 base pair cyclic AMP responsive element from the promoter of the α-subunit gene for the human glycoprotein hormone linked to the herpes simplex virus thymidine kinase promoter in turn linked to CAT (Delegeane et al., (1987) MOL. CELL. BIOL., 7: 3994-4002), and 10 micrograms of the relevant receptor plasmid, using the calcium phosphate precipitation technique (Graham and van der Eb, (1973) VIROLOGY, 52: 456-467). After transfection, cells are maintained in DMEM/5% FCS for 36-40 hours, and then rinsed twice with DMEM. Forskolin (1 µM), a drug known to stimulate adenylate cyclase activity, can then be added in 5 ml DMEM as a positive control, along with the test compounds. Cells are then incubated for 4 hours at 37° C and harvested.

For the CAT assay, after drug incubations cells are rinsed with cold PBS and scraped into 1 ml 40 mM Tris-HCl, pH 7.5, 150 mM NaCl, 1 mM EDTA. Cells are centrifuged and lysed by 3 cycles of freeze-thaw in 200 *µ* l 250 mM Tris-HCl, pH 7.5. [³H]-CAT assays are performed using 50 *µ*l cytosol, 200 nCi [³H]-chloramphenicol and 300 *µ*M butyryl-CoA (Seed and Sheen, (1988) GENE, 67: 271-277). Samples are incubated for 1 hour at 37°C and reactions stopped with the addition of 200 *µ*l mixed xylenes. Butyrylated chloramphenicol is extracted into mixed xylenes which are then back-extracted twice with 200 *µ*l 10 mM Tris-HCl, pH 8.0, 1 mM EDTA. Radiolabeled product is measured by liquid scintillation counting using a Packard Tri-Carb 460C at 50-52% efficiency. Increased CAT activity, indicated by transfer of butyryl groups from butyryl CoA to [3H]-chloramphenicol, is a measure of increased cAMP.

The dosage of the prostaglandin EP₄ agonist component employed in accordance with the present invention varies over a relatively wide range and depends on factors well known in the medicinal arts including, but not limited to: the weight of the individual to whom the agonist component is administered, the general health status/condition of such individual, the disease/condition sought to be treated/prevented by such administration, the severity of such disease/condition in such individual, the potency of the specific EP₄ receptor agonist component being administered, the sensitivity of such individual to the specific agonist component being administered, the mode of administration, the age of such individual, the sex of such individual, the pregnancy status of such individual, the other ongoing drug therapies being administered to such individual and the like factors. The compound (Z)-7-{(1R,4S,5R)-5-[(E)-5-(3-chloro-benzo[b]thiophene-2-yl)-3-hydroxy-pent-1-enyl]-4-hydroxy-3,3-dimethyl-2-oxo-cyclopentyl}-hept-5-enoic acid has an in vitro EC₅₀ (concentration at 50% of maximal efficacy) of between 0.03 and 0.1 nM in both the FLIPR^{®} Ca⁺⁺ flux test and in an alternate activity test measuring changes in the intracellular concentration of cAMP in HEK-EBNA cells expressing the human EP4 prostaglandin receptor.

The amount of prostaglandin EP₄ agonist component employed on a daily basis for each human or animal may be in a range of about 0.1 mg to about 30 mg or about 50 mg or about 100 mg or about 150 mg or about 200 mg or more. In one embodiment, such daily amount may be in a range of about 5 mg to about 150 mg or about 200 mg or more. The prostaglandin EP₄ agonist component may be administered in one or more doses daily, for example, once daily, twice daily, three times daily or more frequently. In one embodiment, once daily dosage is useful.

In general, the prostaglandin EP₄ agonist component is administered for a period of time sufficient to obtain the desired therapeutic effect or effects; that is, relief of the symptoms of the gastrointestinal disorder for which the drug is administered. The duration of treatment may be, for example, in a range of about 1 day or about 3 days or about 1 week or about 2 weeks to about 4 weeks or about 8 weeks or about 12 weeks or about 20 weeks or longer. In one useful embodiment, the duration of treatment is in a range of about 2 weeks to about 12 weeks.

The following non-limiting examples illustrate certain aspects of the present invention.

### EXAMPLES 1 TO 4

A series of four (4) tablet compositions are produced using a prostaglandin EP₄ agonist and three (3) different prostaglandin EP₄ agonist prodrugs. Each of the tablet compositions is prepared as follows.

Within a dust containment area, a mixture of ingredients is prepared and blended until the mixture is uniform. The uniform mixture, having a composition as listed in the table directly below, is then used in a conventional tabletting machine to produce 100 mg tablets having such composition. The tablets may be packaged, for example, in high density polyethylene bottles, with appropriate silica gel packs, capped and labeled.

The mixtures and tablets have the following compositions:

| | **Composition** | | | |
|---|---|---|---|---|
| | 1 | 2 | 3 | 4 |
| Ingredient | Wt. % | wt.% | wt.% | wt.% |
| Prostaglandin EP₄ Agonist 1⁽¹⁾ | 10.0 | - | - | - |
| Prostaglandin EP₄ Agonist Prodrug 1⁽²⁾ | - | 10.0 | - | - |
| Prostaglandin EP₄ Agonist Prodrug 2⁽³⁾ | - | - | 10.0 | - |
| Prostaglandin EP₄ Agonist Prodrug 3⁽⁴⁾ | - | - | - | 10.0 |
| Sugar | 50.0 | 50.0 | 50.0 | 50.0 |
| Excipients⁽⁵⁾ | 40.0 | 40.0 | 40.0 | 40.0 |

(1) 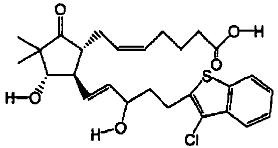
(2) An isopropyl ester of (1) above.
(3) A methyl ester of (1) above.
(4) An amide derivative of (1) above with 2-aminoethanol.
(5) A mixture of conventional pharmaceutical excipients useful, for example as fillers, tabletting aids, bulking agents, preservatives, buffers and the like. Examples include, but are not limited to, mixtures of hydrogenated castor oil, hydroxyl ethyl cellulose, sodium starch glycolate, sorbitol and the like.

Each of the tablets that is produced in Examples 1 to 4 includes about 10 mg of the agonist preparation or prodrug preparation, as the case may be, the total weight of each tablet being about 100 mg.

### EXAMPLES 5 AND 6

A series of two (2) hard shell gelatin capsule compositions are produced using a prostaglandin EP₄ agonist and a prostaglandin EP₄ agonist prodrug. Each of these capsule compositions is prepared as follows.

Within a dust containment area, small sugar spheres are provided. An aqueous mixture of the agonist or prodrug including a binder/sealer, such as Opadry® clear, is provided and is sprayed onto the sugar spheres using a conventional fluid bed spraying system. A second mixture including a binder/sealer, e.g., Opadry® clear, in a liquid carrier is sprayed onto the first sprayed spheres using a conventional fluid bed spraying system. This step results in agonist or prodrug loaded pellets with a sealing coat.

These pellets are coated with an aqueous mixture of triethyl citrate, talc and a methacrylic acid copolymer using a conventional fluid bed spraying system. This step results in agonist or prodrug loaded pellets with a sealing coat and an outer enteric coating. These pellets are encapsulated in natural transparent hard shell gelatin capsules. The filled capsules may be packaged, for example, in high density polyethylene bottles, with appropriate silica gel packs, capped and labeled.

The pellets with the enteric coating have the following compositions.

| | Composition | |
|---|---|---|
| | 5 | 6 |
| Ingredient | wt.% | wt.% |
| Prostaglandin EP₄ Agonist 1⁽¹⁾ | 35.5 | - |
| Prostaglandin EP₄ Agonist Prodrug 2 ⁽²⁾ | - | 35.5 |
| Sugar Spheres | 33.5 | 33.5 |
| Binder/Sealer | 11.0 | 11.0 |
| Methacrylic Acid Copolymer ⁽³⁾ | 14.8 | 14.8 |
| Talc ⁽⁴⁾ | 3.7 | 3.7 |
| Triethyl Citrate ⁽⁵⁾ | 1.5 | 1.5 |

(1) 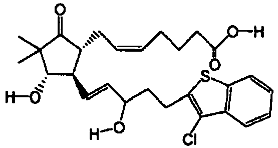
(2) A dextran ester of (1) above.
(3) Enteric coating composition identified as Eudragit® L30-D55 sold by
   Rohm Pharmaceuticals.
(4) Useful as a glidant
(5) Useful as a plasticizer

Each of the capsules that is produced in Examples 5 and 6 includes about 35.5 mg of the agonist or prodrug.

### EXAMPLES 7 TO 10

A series of four (4) soft shell gelatin capsule compositions are produced using a prostaglandin EP₄ agonist and three (3) different prostaglandin EP₄ agonist prodrugs. Each of the capsule compositions is prepared as follows.

Within a dust containment area, a uniform soft shell gelatin mixture, which comprises about 20-45% gelatin, about 15-30% water, about 17.5-35% of a plasticizer which in turn is comprised of glycerin.or sorbitol or a mixture thereof and about 5-25% of a hydrogenated starch hydrolysate, is provided. The soft shell gelatin mixture is agitated with heat until a uniform melt results. A second mixture which is a uniform suspension or solution of the agonist or agonist prodrug is provided as the soft gelatin capsule fill material. The fill material is intended to be substantially water free (less than or equal to about 5-7% water) and includes the agonist or agonist prodrug and optional amounts of co-solvents, buffers, surfactants, thickeners, and the like. The fill material may be of solid, semi-solid, gel, or liquid form, so long as it is uniform and is compatible with soft gelatin encapsulation, i.e., it does not substantially degrade the soft gelatin shell. The fill material has a composition as listed in the table directly below and is preferably degassed prior to use in the next step. The soft shell gelatin preparation above is used to encapsulate 100 mg portions of the fill material employing standard encapsulation technology to produce one-piece, hermetically sealed soft gelatin capsules. The soft shell gelatin capsules may be packaged, for example, in high density polyethylene bottles, with appropriate silica gel packs, capped and labeled.

The fill materials of the capsules have the following make-ups:

### Composition

| | 7 | 8 | 9 | 10 |
|---|---|---|---|---|
| Ingredient | wt.% | wt.% | wt.% | wt.% |
| Prostaglandin EP₄ Agonist 1 ⁽¹⁾ | 10.0 | - | - | - |
| Prostaglandin EP₄ Agonist Prodrug 1⁽²⁾ | - | 10.0 | - | - |
| Prostaglandin EP₄ Agonist Prodrug 2 ⁽³⁾ | - | - | 10.0 | - |

| Prostaglandin EP₄ | | | | |
|---|---|---|---|---|
| Agonist Prodrug 3 ⁽⁴⁾ | - | _ | _ | 10.0 |
| Excipients⁽⁵⁾ | 90.0 | 90.0 | 90.0 | 90.0 |

(1) 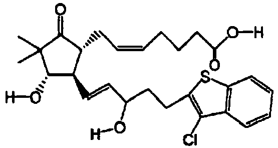
(2) An isopropyl ester of (1) above.
(3) A methyl ester of (1) above.
(4) An amide derivative of (1) above with 2-aminoethanol.
(5) A mixture of optional amounts of conventional pharmaceutical excipients useful, for example, as co-solvents, buffers, preservatives, surfactants, thickeners, and the like, provided they are substantially water free (less than or equal to about 5-7% water) and are compatible with soft gelatin encapsulation, i.e., they do not substantially degrade the soft gelatin shell. Examples include, but are not limited to, mixtures of vegetable oils, liquid polyalkylene glycols and the like.

Each of the capsules that is produced in Examples 7 to 10 includes about 10 mg of the agonist or prodrug, as the case may be, the total weight of each of the capsules depending on the weight of the soft gelatin shell.

### EXAMPLES 11 TO 20

Ten adult humans are diagnosed with gastroesophageal reflux disease. Each of these people orally takes a tablet or a capsule (produced as described in Examples 1 to 10) having a different one of Compositions 1 to 10 once daily for twelve weeks. At the end of this period of time, each of the humans reports substantial relief from the gastroesophageal reflux disease. The pain and/or other symptoms of the disease are reduced.

### EXAMPLES 21 TO 30

Ten adult humans are diagnosed with gastritis. The gastritis is either acute or chronic and includes types such as helicobacter pylori-induced gastritis, atrophic gastritis, pernicious anemia induced gastritis, stress related mucosal damage induced gastritis, alcohol gastropathy induced gastritis, postoperative alkaline gastritis and eosinophilic gastroenteritis. Each of the ten people orally takes a tablet or a capsule (produced as described in Examples 1 to 10) having a different one of Compositions 1 to 10 once daily for twelve weeks. At the end of this period of time, each of the humans reports substantial relief from the gastritis. The pain and/or other symptoms of the disease are reduced.

### EXAMPLES 31 TO 40

Ten adult humans are diagnosed with duodenitis. Each of these people orally takes a tablet or a capsule (produced as described in Examples 1 to 10) having a different one of Compositions 1 to 10 once daily for twelve weeks. At the end of this period of time, each of the humans reports substantial relief from the duodenitis. The pain and/or other symptoms of this disease are reduced.

### EXAMPLES 41 TO 50

Ten adult humans are diagnosed with diverticulitis. Each of these people orally takes a tablet or a capsule (produced as described in Examples 1 to 10) having a different one of Compositions 1 to 10 once daily for twelve weeks. At the end of this period of time, each of the humans reports substantial relief from the diverticulitis. The pain and/or other symptoms of this disease are reduced.

### EXAMPLES 51 TO 60

Ten adult humans are diagnosed with Zollinger-Ellison syndrome. Each of these people orally takes a tablet or a capsule (produced as described in Examples 1 to 10) having a different one of Compositions 1 to 10 once daily for twelve weeks. At the end of this period of time, each of the humans reports substantial relief from the disease. The pain and/or other symptoms of this disease are reduced. In addition, the gastric and/or duodenal ulcers resulting from the disease are reduced in size or substantially completely healed.

### EXAMPLES 61 TO 70

Ten adult humans are diagnosed with chemotherapy induced gastrointestinal toxicity. Each of these people orally takes a tablet or a capsule (produced as described in Examples 1 to 10) having a different one of Compositions 1 to 10 once daily for twelve weeks. At the end of this period of time, each of the humans reports substantial relief from the chemotherapy induced gastrointestinal toxicity. The pain and/or other symptoms arising from the toxicity are reduced.

### EXAMPLES 71 TO 80

Ten adult humans are diagnosed with radiation therapy induced gastrointestinal toxicity. Each of these people orally takes a tablet or a capsule (produced as described in Examples 1 to 10) having a different one of Compositions 1 to 10 once daily for twelve weeks. At the end of this period of time, each of the humans reports substantial relief from the chemotherapy induced gastrointestinal toxicity. The pain and/or other symptoms arising from the toxicity are reduced.

### EXAMPLES 81 TO 90

Ten adult humans have wounds from either injury to and/or surgery of the gastrointestinal tract. Each of these people orally takes a tablet or a capsule (produced as described in Examples 1 to 10) having a different one of Compositions 1 to 10 once daily for twelve weeks. At the end of this period of time, each of the humans reports substantial relief from the pain and/or other symptoms of the injury and/or surgery. In addition, the wounds from the injury/surgery have substantially completely healed.

### EXAMPLES 91-100

Ten adult humans suffer from gastric ulceration. Each of these people orally takes a tablet or a capsule (produced as described in Examples 1 to 10) having a different one of Compositions 1 to 10 once daily for twelve weeks. At the end of this period of time, each of the humans reports substantial relief from the symptoms of the gastric ulcer, including blood loss, lowered hemoglobin and hematocrit volumes. In addition, the ulceration in the stomach has substantially completely healed.

### EXAMPLE 101

The PI3k (phosphoinositide-3-kinase) /Akt phosphorylation pathway is an important cellular signal transduction pathway for the regulation of cell growth, migration, differentiation, and apoptosis. In particular, it has been shown that the PI3k/Akt pathway is essential for the proliferation of intestinal epithelial cells both in *vitro* and in vivo. This pathway transduces proliferative signals between receptors and cell cycle machinery in intestinal epithelial cells. Sheng H., et al., GUT 52:1472-1478 (2003).

Akt (also known as protein kinase B or PKB) is a serine/threonine protein kinase possessing a domain, the PH domain, that binds to phosphatidylinositol (3,4,5)-trisphosphate (PtdIns(3, 4, 5)*P*3, known as PIP₃) with high affinity. PIP₃ is formed by phosphrylation of the di-phosphorylated phosphinositide Ptdins(4, 5)P₂ by activated phosphoinositide-3-kinase, known as PI3k. Once formed the PIP₃ can bind to Akt.

Cycloxygenase (COX) is a family of enzymes (COX 1, COX 2) involved in prostaglandin synthesis. The mechanisms of COX 1 regulation are not entirely well understood. However, considerable work has shown that COX 2 synthesis is at least partly regulated by the PI3k (phosphoinositide-3-kinase)/Akt phosphorylation pathway. Briefly, upon binding PIP₃, Akt is partly activated and is able to be phosphorylated by PDK1 (3-phosphinositide-dependent kinase-1). Upon phosphorylation, Akt is fully activated, and may in turn phosphorylate IκB kinase, which phosphorylates IκB to which is recruited the transcription factors p65 and p50. The phosphorlylated IκB/p65/p50 complex then enters the cell nucleus and stimulates the transcription of COX-2. See e.g., Little et al., J. VET. INTERN. MED. 21:367-377 (2007). The COX-2 inhibitor celecoxib is thought to inhibit the activity of PDK1, therefore also blocking phosphorylation of Akt.

Rat intestinal epithelial cells (IEC-18 cells) are immortalized normal epithelial cells purchased from the American Type Culture Collection (ATCC). For these experiments, IEC-18 cells were cultured in high glucose Delbecco's Modified Eagle's Medium (DMEM) at 37°C under 5% CO₂ and incubated for 24 hours with either a) 5 µM of the COX-1 inhibitor SC-560 (purchased from Sigma-Aldrich, St. Louis, MO), 10 µM of the COX-2 inhibitor celecoxib, or 10 µM of the COX-1/2 inhibitor indomethacin. Drugs were provided in a 0.1% dimethylsulfoxide (DMSO) vehicle.

The PI3k/Akt pathway is monitored using the following general assay procedure. Following incubation cell lysates are collected with lysis buffer containing phosphatase and proteinase inhibitors. The supernanats are collected and protein concentrations are measured. Then SDS(sodium dodecyl sulfate) sample buffers are made to make sure final protein concentration is equal in each sample and boiled for 10 minutes at 100°C. Then the samples are loaded on to a 4%-12% pre-made SDS-PAGE (polyacrylamide gel electrophoresis) and the gel is developed under electrophoresis. When the samples reach the bottom of the gel, the gel is removed from the electrophoresis equipment and transferred to a nitrocellulose membrane and the membrane blocked with milk. The membrane is incubated with a primary antibody selective for either a) total Akt or b) phosphrylated Akt overnight at 4°C. A secondary antibody having a conjugated horseradish peroxidase (HRP) moiety is used to bind the constant regions of the primary antibodies. The membrane is washed under conditions favoring selective antibody binding. Finally, the HRP is detected using a enzymatically initiated chemiluminescent substrate such as Super Signal@ ELISA Pico Substrates, available from Thermo Fisher Scientific.

As shown in Fig. 1, each of these agents inhibited Akt phosphorylation. Specifically, indomethacin inhibited phosphorylation of Akt to a greater extent that did SC-560 or celecoxib under these conditions. However, co-incubation of the cells for 24 hours with both 10 µM indomethacin and 10nM of the prostaglandin EP4 agonist of Composition 7 (hereinafter "Compound 7") maintained Akt phosphorylation at higher levels compared to those samples provided indomethacin in the absence of added EP4 agonist. Therefore, prostaglandin EP4 agonists such as Compound 7 may restore or strengthen Akt activity, either when used alone, or in the presence of COX inhibitors, such as non-steroidal anti-inflammatory drugs (NSAIDS).

### EXAMPLE 102

IEC-18 cells were cultured in high glucose DMEM containing 10% fetal bovine serum (FBS), 1% penicillin, 1% streptomycin, and 50 unit insulin essentially as indicated in Example 101 for 48 hours in the presence of the 0.05 µg/ml chemotherapy agent doxorubicin to induce apoptosis. In the presence of 10 µM celecoxib (which inhibits endogenous prostaglandin PGE2 expression), apoptosis of doxorubicin-treated IEC-18 cells proceeds within 48 hours at such very low concentrations of doxorubicin. Finally, the cells collected by trypsin were subjected to flow cytometry analysis for identification of the percentage of cells in different stages of the cell cycle.

Cultured cells were prepared for flow cytometry by trypsinization and centrifugation. The cells were then washed with cold phosphate buffered saline solution (PBS), then fixed with cold 70% ethanol overnight. The cells were then again washed with PBS and stained with propidium iodide, a nucleic acid intercalating dye, in the presence of RNase for 30 minutes at room temperature. The cells were then sorted using the buffer provided by Becton-Dickenson, the maker of the flow cytometry equipment.

Fig. 2A shows florescent flow cytometry analysis of IEC-18 cells in vehicle alone; Fig. 2B shows flow cytometry analysis under the same conditions (except that the cells have previously been treated with 0.05 µg/ml doxorubicin (D) and 10 µM celecoxib (C); Fig. 2C shows the results when IEC-18 cells are incubated with 0.05 µg/ml doxorubicin (D), 10 µM celecoxib (C) and 10 nM Compound 7.

Additionally, using CellQuest^{®} software (Becton-Dickenson) a quantification of the percent of apoptosis occurring in IEC-18 cells after 48 hours' incubation was performed. Fig 2D shows the percent apoptosis of cells in 0.1% DMSO vehicle alone is approximately 2%. By contrast, the percentage of cells undergoing apoptosis increases to about 13% upon treatment with doxorubicin and 10µM celecoxib. However, addition of 10 nM Compound 7 to the incubation mixture results in a reduction of apoptosis to approximately 5%, close to the baseline level.

Thus, this experiment shows that Compound 7, a prostaglandin EP4 agonist, reduces the cytotoxic effect of NSAIDS and chemotherapeutic agents upon intestinal epithelial cells, thus providing a protective effect against injury to the gut caused by such agents.

### EXAMPLE 103

Gastric ulcers were produced in C57BL/6 mice by using 40% acetic acid as follows. Animals were anesthetized. The abdomen of each animal was surgically opened, and the stomach exposed. A 3 mm curette was placed onto the anterior surface of each stomach. A solution of 40% acetic acid was placed inside the curette. The curette prevents damage to surrounding tissue. The acetic acid treatment causes damage to blood vessels of the stomach, producing chemical death to the mucous layer.

On the third day following induction of ulceration, the animals were given various treatments by oral gavage. First, vehicle (4% DMSO-corn oil) or a low dose (3 mg/kg/day) of indomethacin in 4% DMSO-corn oil was used. This dose of indomethacin has been reported not to induce gross lesions on the stomach, although it can inhibit the production of endogenous prostglandin PGE2.

On day 7 blood was withdrawn for hematology analysis from sacrificed animals, and subjected to automated testing for standard hematological parameters. Ulcer areas (defined as the regions of the stomach not covered by stomach mucosal epithelial cells) were measured by dissection microscopy for macroscopic analysis; additionally, the widest section of each ulcer sample was measured and observed as the size index under microscopy. The stomachs were processed for pathological analysis by dissection and section through the widest part of the ulcer. Sections were stained using hematoxylin & eosin.

The results indicated that indomethacin delayed the spontaneous healing of gastric ulcers using both gross and microscopic measurements (Fig. 3A), exacerbated inflammation (Fig. 3B; indicated by an increase in lymphocytes (LYM) in the blood). Fig. 3B shows an x-axis legend of "WBC" (white blood cells), LYM (lymphocytes; an increase in which is a standard indicator of chronic inflammation), NEU (neutrophils) and MONO(monocytes). The latter two cell types are associated with the acute (for example, bacterially-mediated) immune response. WBC therefore is a measurement of all white blood cells (including lymphocytes, neutrophils and monocytes), with the particular cell types then "broken out" in the following bars. Indomethacin was found to exacerbate blood loss at ulcer sites (Fig. 3C). The above results indicate that endogenous PGE2 play an important role in promoting the spontaneous healing of chronic gastric ulcer. Hematological profiles were analyzed by persons blinded to treatments using automatic ADVIA120 Hematology System (Bayer, Tarrytown, NY).

Mice were then treated with 10 nM Compound 7 or the vehicle under otherwise identical conditions. In this experiment the vehicle was 4% DMSO in corn oil in a volume of 0.1 ml. The mice were sacrificed and the stomachs examined on either the 7^{th} day or the 11^{th} day following ulceration. The results indicate that mice treated with 10 nM Compound 7 resulted in significantly smaller ulcer sizes than mice treated with vehicle (Fig. 4A). Under dissection microscopy, the areas of the ulcers in mice treated with Compound 7 were 60% and 32% of the control on days 7 and 11, respectively. The ulcers from mice treated with Compound 7 had significantly less inflammatory cell infiltrations and necrotic tissue in granulation tissue compared to those from the control group, which may indicate a healthier condition for re-epithelization. (Fig. 4B).

Fig. 4C shows the representative gross and microscopic morphologies of the ulcer tissue in this experiment taken after sacrificing the mice treated with the vehicle on day 7 and the same vehicle containing Compound 7, respectively. As can be seen, the photographic results correlate with the pathology results and demonstrate that the prostaglandin EP4 agonist Compound 7 stimulates healing of ulcer and facilitate the removal of necrosis tissue.

As a further indication of the effect of prostaglandin EP4 agonists on NSAID-induced gastrointestinal tissue damage, hematology indicated that mice treated with both 0.1 mg/kg/d Compound 7 and 3 mg/kg/d indomethacin had significantly less blood loss than those mice in which ulcers were induced and were subsequently treated with indomethacin alone. Fig. 5A shows that the number of red blood cells ("RBC") x 10⁶/µl of whole blood is significantly greater in mice administered Compound 7 ("treated mice") as compared to those given the vehicle alone ("control mice"). In this experiment the data from 20 treated mice were averaged (n=20) and compared to the averaged data from 20 control mice. Similarly, in both treated and control populations of animals both the amounts of hemoglobin (HGB), expressed in grams per decaliter, and hematocrit (HCT), expressed in % blood volume, was determined. Both HGB and HCT were significantly greater in the treated mice than the control mice; these data, like the low RBC count, are typical indicia of blood loss in the gastrointestinal tract.

Fig. 5B shows that the treated group had significantly lessened inflammatory response than did the control group of mice. Thus, WBC and lymphocyte counts were significantly higher in the control group than in the treated group, while the level of neutrophils in the blood remains relatively constant.

Thus, the data clearly show that prostaglandin EP4 agonists such as Compound 7 are able to cause an acceleration in the healing of stomach ulcers. Additionally, such agents are able to prevent or moderate the cytotoxic effects of NSAIDS (for example, indomethacin) in the gastrointestinal tract, particularly preventing or moderating the extent of apoptosis in intestinal and gastric epithelial cells.

Accordingly, the present invention also envisions a therapeutic composition comprising a prostaglandin EP₄ agonist component having a structure: 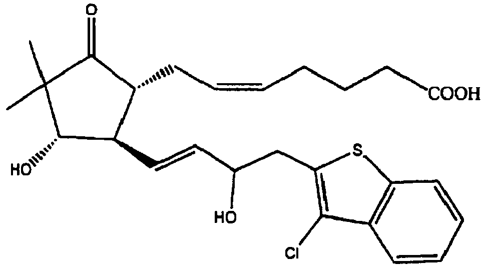 in combination with a non-steroidal anti-inflammatory drug (NSAID). The NSAID may be any NSAID, but in particular may be selected from the group consisting of aspirin, acetomenifen, indomethacin, ibuprofen, ketorolac, napoxen, piroxicam, nabumetone, celecoxib, diclofenac, diflunisal, etodolac, fenoprofen, ketoprofen, mefenamic acid, meloxicam, nabumetone, oxaprozin, sulindac, and tolmetin.

In addition the present invention may comprise a method of treating a patient having a condition responsive to treatment by an NSAID comprising administering to said patient an NSAID and a prostaglandin EP₄ agonist component comprising a structure:

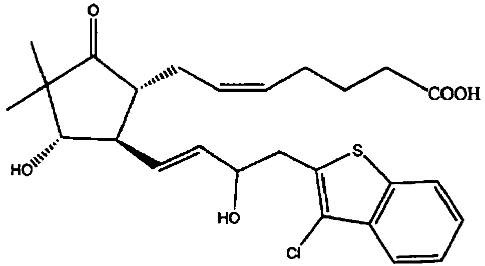

Of course, the prostaglandin EP4 agonist component may comprise a prodrug of the compound corresponding to said structure.

Each and every reference, article, patent, patent application and publication cited and/or set forth above is hereby incorporated herein by reference in its entirety. While this invention has been described with respect to various specific examples and embodiments, it is to be understood that the invention is not limited thereto and that it can be variously practiced within the scope of the following claims.

## Claims

1. A prostaglandin EP₄ agonist component comprising a structure: 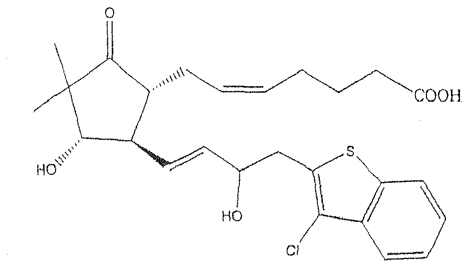 or a pharmaceutically acceptable salt of said prostaglandin EP₄ agonist, a pro-drug of said prostaglandin EP₄ agonist or a mixture of two or more of these agents, for use in the treatment of a disease or a condition selected from the group consisting of gastroesophageal reflux disease, acute and chronic gastritis, duodenitis, diverticulitis, Zolliger-Ellison syndrome, chemotherapy-induced gastrointestinal toxicity, radiation-induced gastrointestinal toxicity, NSAID-induced gastrointestinal toxicity, gastric ulcers, duodenal ulcers, intestinal ulcers and wounds or damage to the gastrointestinal tract caused by injury or surgery, thereby treating or preventing the disease or condition.

2. Use of a prostaglandin EP₄ agonist component comprising a structure: 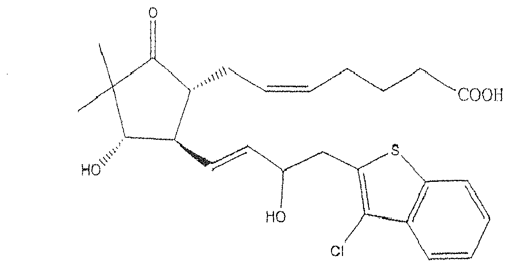 or a pharmaceutically acceptable salt of said prostaglandin EP₄ agonist, a pro-drug of said prostaglandin EP₄ agonist or a mixture of two or more of these agents, in the preparation of a therapeutic agent for the treatment of a disease or condition selected from the group consisting of gastroesophageal reflux disease, acute and chronic gastritis, duodenitis, diverticulitis, Zolliger-Ellison syndrome, chemotherapy-induced gastrointestinal toxicity, radiation-induced gastrointestinal toxicity, NSAID-induced gastrointestinal toxicity, gastric ulcers, duodenal ulcers, intestinal ulcers and wounds or damage to the gastrointestinal tract caused by injury or surgery, thereby treating or preventing the disease or condition.

3. Compound or use according to any of claim 1 or 2, wherein the pro-drug is an ester, ether, or amide of a carbohydrate; or the pro-drug is an ester, ether, or amide of an amino acid.

4. The compound or use according to any of claims 1 to 3, wherein the prostaglandin EP₄ agonist component comprises a glucoside ester, ether, or amide; a glucuronide ester, ether, or amide; a cyclodextrin ester, ether, or amide; or a dextran ester, ether, or amide.

5. The compound or use according to any of claims 1 to 4, wherein said disease or condition is a gastritis selected from the group consisting *Heliobacter* pylori-induced gastritis, atrophic gastritis, pernicious anemia, stress-related mucosal damage, alcohol gastropathy, gastric ulcers, duodenal ulcers, intestinal ulcers, postoperative alkaline gastritis, and eosinophilic gastroenteritis.

6. The compound or use according to any of claims 1 to 5, for the treatment of a mammal in need of treatment for said condition or disease.

7. The compound or use according to claim 6, for the treatment of a mammal in need of treatment for said condition or disease.

8. Compound or use according to any one of claims 1 to 7, wherein the compound is administered to a gastrointestinal tract of the mammal.

9. A therapeutic composition comprising an NSAID and a prostaglandin EP₄ agonist component comprising a structure: 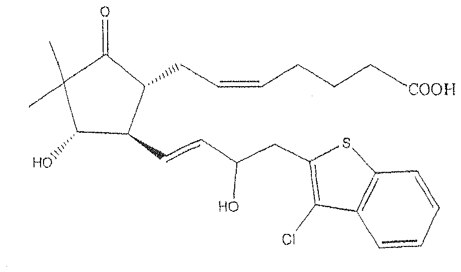 or a pharmaceutically acceptable salt of said prostaglandin EP₄ agonist, a pro-drug of said prostaglandin EP₄ agonist or a mixture of two or more of these agents.

10. The composition according to claim 9, wherein the pro-drug is an ester, ether, or amide of a carbohydrate; or the pro-drug is an ester, ether, or amide of an amino acid.

11. The composition according to claim 9 or 10, wherein the prostaglandin EP₄ agonist component comprises a glucoside ester, ether, or amide; a glucuronide ester, ether, or amide; a cyclodextrin ester, ether, or amide; or a dextran ester, ether, or amide.

12. The composition according to any of claims 9 to 11 wherein the NSAID is selected from the group consisting of aspirin, acetomenifen, indomethacin, ibuprofen, ketorolac, napoxen, piroxicam, nabumetone, celecoxib, diclofenac, diflunisal, etodolac, fenoprofen, ketoprofen, mefenamic acid, meloxicam, nabumetone, oxaprozin, sulindac, and tolmetin.
